# EUROPÄISCHE PATENTANMELDUNG

(11) **EP 1 764 039 A1**
(43) Veröffentlichungstag der Anmeldung: **21.03.2007**
(21) Anmeldenummer: 06119982.4
(22) Anmeldetag: 01.09.2006
(51) Int. Cl.: A61B 6/00, A61N 5/00, A61B 6/04, A61N 5/10, A61B 6/03

(54) **Verfahren und Vorrichtung zur Untersuchung und Behandlung eines Patienten**

(30) Priorität: 16.09.2005 DE 102005044415; 16.09.2005 US 717832 P
(71) Anmelder: SIEMENS AKTIENGESELLSCHAFT, 80333 München (DE)
(72) Erfinder: Herrmann, Klaus, 90403 Nürnberg (DE); Rietzel, Eike, 64289 Darmstadt (DE); Sommer, Andres, 91094 Langensendelbach (DE)

(57) **Zusammenfassung**

Verfahren zur Untersuchung und Behandlung eines Patienten (2), bei dem der Patient (2) während einer Untersuchung auf eine erste Patientenliege (4) positioniert wird, wo eine Untersuchung mit einem Bild gebenden Untersuchungsgerät (6, 14), insbesondere mit einem Computertomographen (6), durchgeführt wird. Zur Behandlung wird der Patient (2) auf eine Patientenliege (4') positioniert, die eine zur ersten Patientenliege (4) identisch aufgebaute Liegefläche (10, 10') und Positionsmechanik (12, 12') aufweist. Hierbei kann zur Untersuchung und zur Behandlung dieselbe Patientenliege (4, 4') verwendet werden, oder der Patient (2) wird zur Untersuchung auf die erste Patientenliege (4) und zur Behandlung auf eine zweite separate Patientenliege (4') positioniert, wobei beide Patientenliegen (4, 4') identische Positionsmechanik (12, 12') aufweisen.

## Beschreibung

Die Erfindung betrifft ein Verfahren zur Untersuchung und Behandlung eines Patienten. Die Erfindung betrifft weiterhin einen Patientenraum und ein Untersuchungsgerät.

Für gewöhnlich wird vor Durchführung einer Strahlentherapie das zu bestrahlende Körpergebiet eines Patienten mit einem Bild gebenden Diagnosegerät, insbesondere mit einem Computertomographen (CT), untersucht. Um möglichst genaue Aufnahmen mit einer guten Auflösung zu erhalten, wird der Patient auf einer Patientenliege immobilisiert und die Patientenliege wird zumindest teilweise durch das Untersuchungsgerät gefahren. Mit Hilfe der hierdurch erstellten Aufnahmen wird ein Datensatz gewonnen, mittels dessen ein Bestrahlungsplan ausgearbeitet wird, nach dem der Patient in einer oder in mehreren Sitzungen der Strahlentherapie ausgesetzt wird. Die Strahlentherapie findet in der Regel in einem separaten Zimmer statt, in welchem eine Bestrahlungsvorrichtung, wie z.B. ein Linearbeschleuniger, aufgestellt ist. Der Patient muss hierbei vor der Strahlentherapie auf eine zweite Patientenliege repositioniert werden, die zur Bestrahlungsvorrichtung orientiert ist, und der Patient soll die gleiche Körperstellung annehmen.

Eine gewünschte Orientierung der zweiten Patientenliege zur Bestrahlungsvorrichtung und eine gleiche Körperstellung des Patienten auf beiden Patientenliegen wird insbesondere durch die Verwendung eines Computertomographen als Untersuchungsgerät erschwert. Damit die Bild gebende Einheit eines Computertomographen möglichst dicht um den Patienten fahren kann, muss die kreisförmige Öffnung des Computertomographen möglichst klein gehalten werden. Dies wird durch die spezielle Form einer gewöhnlichen CT-Patientenliege erreicht: sie ist weniger breit als eine Patientenliege einer Bestrahlungsvorrichtung, ist verhältnismäßig dünn und weist eine Hohlkehle auf, in welcher der Patient bei der Untersuchung gelagert wird.

Die Hohlkehle ist für die Diagnose und die Strahlentherapie unvorteilhaft, weswegen sie mit einer eine konvexe Seite aufweisenden Einlage, die beispielsweise aus der deutschen Gebrauchsmusterschrift DE 201 21 050 U1 zu entnehmen ist, aufgefüllt wird. Erst dann wird eine Aufnahme für die Strahlentherapieplanung gemacht.

Weiter biegt sich die Patientenliege des Computertomographen aufgrund ihrer mangelnden Stärke beim Einfahren in den Computertomographen je nach Scanposition unterschiedlich weit durch, so dass bei den Bildaufnahmen geometrische Verzerrungen entstehen können. Wenn durch die zweite Patientenliege bestrahlt werden soll, ist eine übliche Vorgehensweise, die erste Patientenliege aus dem mittels der CT-Aufnahmen gewonnenen Datensatz zu eliminieren und durch die Daten über die zweite Patientenliege, die bei der Strahlentherapie verwendet wird, zu ergänzen.

Der Erfindung liegt die Aufgabe zugrunde, ein Verfahren zur Untersuchung und Behandlung eines Patienten anzugeben, mit dessen Hilfe die Schwierigkeiten, die bei der Planung und Durchführung einer Untersuchung und einer anschließenden Strahlentherapie entstehen, in einfacher Weise beseitigt werden. Weiterhin liegt der Erfindung die Aufgabe zugrunde, einen Patientenraum sowie ein Untersuchungsgerät anzugeben, mittels deren das Verfahren durchgeführt werden kann.

Die auf ein Verfahren gerichtete Aufgabe wird erfindungsgemäß gelöst durch ein Verfahren zur Untersuchung und Behandlung eines Patienten, bei dem:
a) der Patient zu einer Untersuchung auf eine erste Patientenliege positioniert wird, wo
b) eine Untersuchung eines zu behandelnden Gewebes mit einem Bild gebenden Untersuchungsgerät, insbesondere mit einem Computertomographen, durchgeführt wird, und
c) der Patient zur Behandlung auf eine Patientenliege positioniert wird, die eine zur ersten Patientenliege identisch aufgebaute Liegefläche aufweist, wo
d) das zu behandelnde Gewebe mit einer Bestrahlungsvorrichtung bestrahlt wird.

Der wesentliche Vorteil dieses Verfahrens ist darin zu sehen, dass eine sehr hohe Präzision bei gleichzeitiger Vereinfachung des gesamten Behandlungsprozesses gewährleistet ist. Durch den Einsatz von Patientenliegen mit identisch aufgebauten Liegeflächen bei der Untersuchung und bei der Behandlung kann eine Korrektur der Patientenliege in der Strahlentherapieplanung entfallen. Durch die gleichen mechanischen Eigenschaften identisch aufgebauter Liegeflächen spielen außerdem störende Biegeeffekte, die durch die Form und den Aufbau der Patientenliege entstehen, keine Rolle mehr. Die während der Untersuchung mit dem Bild gebenden Untersuchungsgerät gemessenen Grauwerte und die Information über die geometrische Form der Liegefläche werden gespeichert. Da die auf die Geometrie der Patientenliege bezogenen Parameter wegen des identischen Aufbaus der Liegeflächen beider Patientenliegen nicht unterschiedlich sind, muss die bei der Untersuchung gewonnene Information über die Patientenliege nicht wie bisher aus dem Datensatz der Untersuchung gelöscht und durch weitere Daten ersetzt werden. Somit kann die Information sowohl über die Lage des Patienten als auch über die Patientenliege bei der Bestrahlung einfach weiter verwendet werden, was dieses Verfahren deutlich einfacher gegenüber der gewöhnlichen Vorgehensweise macht. Weiterhin werden durch den Einsatz identisch aufgebauter Liegeflächen Probleme bei der Immobilisierung des Patienten vermieden, die durch die bisher unterschiedlichen Liegeflächen bei der Untersuchung und der Behandlung entstanden waren.

Damit auch die Unterschiede der mechanischen Stabilität der Untersuchungs-Patientenliege und der Behandlungs-Patientenliege ebenfalls eliminiert werden, wird vorzugsweise der Patient zur Behandlung auf einer Patientenliege positioniert, die eine zur ersten Patientenliege identisch aufgebaute Positionsmechanik aufweist. Unter Positionsmechanik wird im einfachsten Fall ein Sockel verstanden, auf den die Patientenliege gestützt ist. Eine kompliziertere Positionsmechanik umfasst mehrere bewegliche Komponenten, mit deren Hilfe die Patientenliege verstellt oder verfahren werden kann.

Bevorzugt wird zur Untersuchung und zur Behandlung dieselbe Patientenliege verwendet. Dies stellt die einfachste und kostengünstigste Ausführung des Verfahrens dar. Bei einer Ausführung wird die Patientenliege nicht bewegt, sondern das Untersuchungsgerät und / oder die Bestrahlungsvorrichtung werden über sie gefahren. Diese Ausführung bietet die höchste Präzision bei der Planung und Bestrahlung des zu behandelnden Gewebes, denn der Patient muss lediglich ein mal auf der Patientenliege zur Untersuchung immobilisiert werden und zur Behandlung muss er weder auf eine andere Patientenliege repositioniert noch zu einem anderen Ort gebracht werden.

Wenn das Untersuchungsgerät und die Bestrahlungsvorrichtung nicht derart ausgelegt sind, dass sie beide über dieselbe unbewegliche Patientenliege gefahren werden können, wird in einer anderen bevorzugten Ausführung die Patientenliege nach der Untersuchung zur Bestrahlungsvorrichtung hin verfahren. Diese Ausführung ist ebenso kostengünstig und bietet eine sehr hohe Genauigkeit bei der Planung und Behandlung, denn eine Repositionierung des Patienten auf eine weitere Liege ist nicht erforderlich. Es werden lediglich die Ortskoordinaten der Patientenliege innerhalb eines Patientenraums geändert, wobei das zu behandelnde Gewebe zur Bestrahlungsvorrichtung hin gleich wie zum Untersuchungsgerät orientiert wird. Die Patientenliege kann eventuell in senkrechter Richtung verstellt werden.

In sehr vielen Fällen verhält es sich jedoch so, dass die Untersuchung und die Behandlung eines Patienten in zwei getrennten Zimmern stattfinden, welche möglicherweise weit entfernt voneinander liegen. Auch ist es vorstellbar, dass die Untersuchungs- und Behandlungsgeräte so aufgebaut sind, dass die dazugehörigen Patientenliegen untrennbar mit den Geräten verbunden sind, so dass ein Verfahren der Patientenliege von einem zum anderen Gerät sehr schwierig oder gar unmöglich ist. Deswegen wird der Patient in einer weiteren bevorzugten Ausgestaltung zur Untersuchung auf die erste und zur Behandlung auf eine zweite separate Patientenliege positioniert. Die beiden separaten Patientenliegen weisen die gleichen Liegeflächen auf, so dass ein Ausgleich der Liegeflächen mittels Einlagen, wie es üblicherweise im bekannten Stand der Technik gemacht wird, nicht mehr notwendig ist. Hiermit werden die gleichen Vorteile wie beim Verwenden derselben Patientenliege für die Diagnose und für die Strahlentherapie erreicht.

Vorzugsweise werden die erste und die zweite Patientenliege jeweils über eine identische Positionsmechanik zur Untersuchung und zur Behandlung in gleichen Stellungen verstellt. Durch die identische Positionsmechanik wird gleiche Einstellgenauigkeit bei beiden Patientenliegen erzielt. Hiermit können außerdem Biegeeffekte, die durch die Form und Aufbau der Patientenliegen entstehen, vollkommen ignoriert werden. Die Liegefläche einer herkömmlichen Patientenliege für einen Computertomographen ist z.B. sehr dünn und biegt sich unter dem Gewicht des Patienten durch. Dieser Effekt kann mittels Verwendung von identischen Patientenliegen mit identischer Positionsmechanik nicht völlig eliminiert werden, aber beim Verstellen der zwei identischen Patientenliegen ist dieser Effekt, soweit er vorliegt, bei der Untersuchung und bei der Behandlung gleich groß und muss deswegen bei der Strahlentherapieplanung und beim Behandlungsprozess nicht berücksichtigt werden.

Gemäß einer bevorzugten Ausgestaltung wird die räumliche Stellung der ersten Patientenliege von einer Recheneinheit in einem Datensatz erfasst und dieser Datensatz wird für eine automatische Einstellung der zweiten Patientenliege über ihre Positionsmechanik verwendet. Somit wird ein hoher Automatisierungsgrad des Verfahrens erreicht, wobei die zwei Patientenliegen mit einer sehr hohen Genauigkeit in die gleiche Stellung gebracht werden.

Zweckdienlicherweise wird die Behandlung mit der Bestrahlungsvorrichtung in einem anderen Zimmer als die Untersuchung mit dem Untersuchungsgerät durchgeführt. Durch den Einsatz von identischen Patientenliegen, d.h. identische Liegefläche und Positionsmechanik, können hierbei die oben genannten Vorteile gewonnen werden, auch im Falle, dass die zwei Zimmer einen großen räumlichen Abstand voneinander aufweisen, wie z.B. wenn die zwei Zimmer sich in unterschiedlichen Gebäuden oder sogar in unterschiedlichen geographischen Orten befinden.

Vorteilhafterweise wird die Liegefläche der Patientenliege in einen Bestrahlungsplan bei der Bestrahlung des zu behandelnden Gewebes einbezogen. Da die Daten über die erste Patientenliege nicht mehr ersetzt werden, wird die Ausarbeitung des Bestrahlungsplans wesentlich schneller und einfacher, insbesondere wenn durch die Liegefläche gestrahlt werden soll.

Die zweitgenannte Aufgabe wird erfindungsgemäß gelöst durch einen Patientenraum mit einer Untersuchungsstelle, an der ein Untersuchungsgerät, insbesondere ein Computertomograph aufgestellt ist, und einer Behandlungsstelle, an der eine Bestrahlungsvorrichtung aufgestellt ist, wobei an dem Untersuchungsgerät und an der Bestrahlungsvorrichtung Patientenliegen mit identisch aufgebauten Liegeflächen positionierbar sind. Die in den vorhergehenden Ansprüchen angegebenen Vorteile sind sinngemäß auf diesen Patientenraum zu übertragen. Unter Patientenraum kann dabei ein großes Zimmer, eine Krankenhausabteilung, eine Arztpraxis, eine Klinik oder eine andere Vorrichtung mit einer Diagnostik- und/oder einer Behandlungsstelle an sich oder in Kombination verstanden werden.

Bevorzugt weist eine Patientenliege eine Positionsmechanik auf, mit deren Hilfe die Patientenliege vom Untersuchungsgerät zur Behandlungsvorrichtung bewegbar ist.

Zur Lösung der letztgenannten Aufgabe wird erfindungsgemäß ein Untersuchungsgerät zur Durchführung einer medizinischen Untersuchung eines Patienten angegeben, an dem eine Patientenliege positionierbar ist, deren Liegefläche zur Positionierung an einer Bestrahlungsvorrichtung ausgebildet ist.

Mit dieser Ausgestaltung wird erzielt, dass dieselbe Patientenliege sowohl am Untersuchungsgerät als auch an der Behandlungsvorrichtung verwendet werden kann. Dabei kann die Patientenliege sowohl beweglich als auch unbeweglich ausgebildet sein. In beiden Fällen muss sie aber trennbar vom Untersuchungsgerät ausgestaltet sein. Eine Variante des Untersuchungsgeräts sieht vor, dass die bewegliche Patientenliege nach der Untersuchung einfach zur Behandlungsvorrichtung hingefahren wird. Gemäß einer anderen Variante ist die Patientenliege unbeweglich und das Untersuchungsgerät wird zur Diagnostik über die Patientenliege hergefahren und nachher weggefahren. Die Bestrahlungsvorrichtung kann ebenfalls verstellbar sein, auch wenn sie an einer Stelle an einer Wand oder an einer Decke des Raums befestigt ist.

Ausführungsbeispiele der Erfindung werden anhand einer Zeichnung näher erläutert. Es zeigen hierbei schematisch und teilweise vereinfacht:
- FIG 1: eine Patientenliege mit einem darauf positionierten Patienten während einer Untersuchung mit einem Computertomographen,
- FIG 2: eine Patientenliege gemäß FIG 1 mit einem darauf positionierten Patienten während einer Untersuchung mit einem weiteren Bild gebenden Untersuchungsgerät, und
- FIG 3: eine Patientenliege gemäß FIG 1 mit einem darauf positionierten Patienten während einer Bestrahlung mit einer Fixed-Beam-Bestrahlungsvorrichtung.

Gleiche Bezugszeichen haben in den verschiedenen Figuren die gleiche Bedeutung.

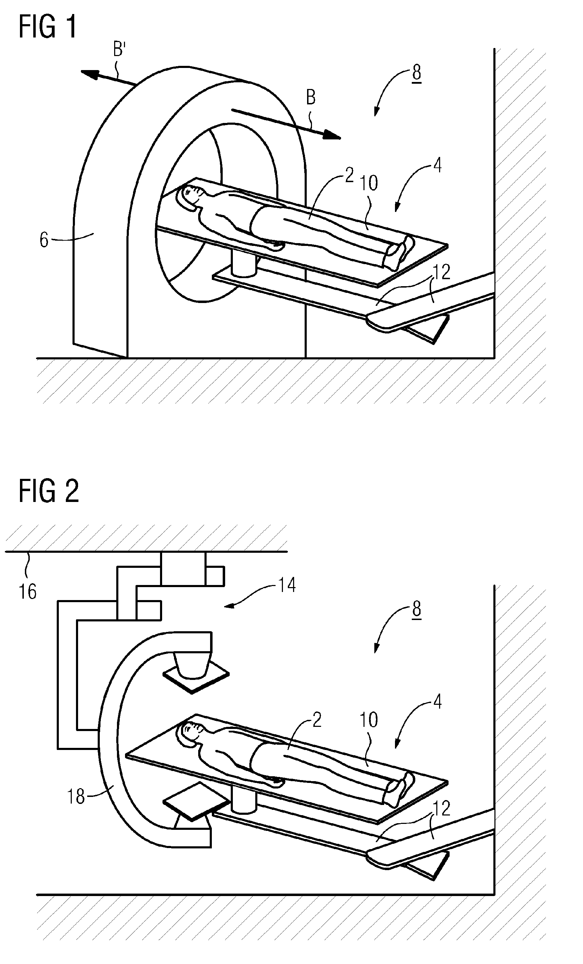

FIG 1 zeigt schematisch einen Patienten 2, der auf einer Patientenliege 4 zur Untersuchung positioniert und immobilisiert ist. Die Untersuchung wird mit einem Bild gebenden Untersuchungsgerät 6, in diesem Fall ein Computertomograph, durchgeführt. Die Patientenliege 4 und der Computertomograph 6 befinden sich in einem Patientenraum 8.

Die Patientenliege 4 weist eine Liegefläche 10 und eine Positionsmechanik 12 auf, welche in einer bestimmten Stellung gebracht sind. Die Positionsmechanik 12 kann viele unterschiedliche Komponenten umfassen und ist somit unterschiedlich kompliziert aufgebaut. Im einfachsten Fall, wenn die Patientenliege 4 unbeweglich ausgestaltet ist, umfasst die Positionsmechanik 12 beispielsweise einen stationären Sockel. In komplizierteren Ausführungsformen weist die Positionsmechanik 12 mehrere Komponenten auf, die beweglich miteinander verbunden sind und ein Verstellen der Patientenliege 4 in allen Richtungen sowie ein Verfahren der Patientenliege 4 zu einem anderen Ort erlauben.

Die Liegefläche 10 umfasst hierbei den Bereich der Patientenliege 4, auf dem der Patient 2 gelagert wird und ist nach Art einer dünnen Platte ausgebildet.

Weiterhin weist die Patientenliege 4 bestehend aus der Liegefläche 10 und der Positionsmechanik 12 nicht dargestellte Sensoren auf, die die räumliche Stellung der Liegefläche 10 und / oder der Positionsmechanik 12 erfassen und an eine Recheneinheit senden, so dass die Information in einem Datensatz gespeichert werden kann. Dieser Datensatz wird später verwendet, um eine Patientenliege 4', auf welcher der Patient 2 zur Behandlung positioniert ist, automatisch in die genau gleiche Stellung einzustellen.

Der Computertomograph 6 ist derart ausgestaltet, dass seine kreisförmige Öffnung um die Liegefläche 10 der Patientenliege 4 verfahrbar ist, was mit den Pfeilen B und B' angedeutet ist. Mittels des Computertomographen 6 werden Aufnahmen von Gebieten am oder im Körper des Patienten 2 und von der Liegefläche 10 gemacht. Während der Untersuchung wird Information über die Geometrie, Abmessungen und Orientierung der Patientenliege 4 erhalten und im Datensatz gespeichert, wobei zumindest ein Teil dieser Information aus den Grauwerten der CT-Aufnahmen zu entnehmen ist. Diese Information wird zusammen mit den Daten über die genaue Positionierung des Patienten 2 bei der Strahlentherapieplanung verwendet.

FIG 2 zeigt die Patientenliege 4 gemäß FIG 1 zur Lagerung eines Patienten 2 während einer Untersuchung im Raum 8 mit einem alternativen Bild gebenden Untersuchungsgerät 14. Im Gegensatz zum Computertomographen 6 ist das Untersuchungsgerät 14 an der Raumdecke 16 befestigt. Das Untersuchungsgerät 14 weist einen mehrkomponentigen Aufbau auf, durch den eine C-förmige Untersuchungseinheit 18 in alle Richtungen verstellt werden kann, um Aufnahmen von einem zu behandelnden Gewebe zu erstellen.

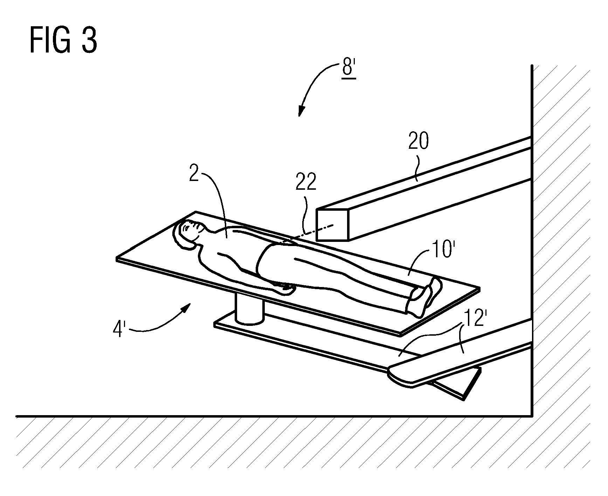

In FIG 3 ist eine Patientenliege 4' zur Lagerung eines Patienten 2 während einer Behandlung in einem Patientenraum 8' dargestellt. Die Patientenliege 4' weist einen identischen Aufbau der Liegefläche 10' und der Positionsmechanik 12' wie die Patientenliege 4 gemäß FIG 1 und FIG 2 auf. Die Patientenliegen 4 und 4' können sogar ein und dieselbe Patientenliege sein. Ebenfalls gilt, dass die Patientenräume 8 und 8' ein und derselbe sein können, insbesondere wenn die Patientenliege 4, 4' ortsfest ist und eine eingeschränkte Bewegungsfreiheit aufweist. Allerdings ist es auch möglich, dass die beiden Patientenräume 8 und 8' zwei separate Zimmer sind, die einen großen räumlichen Abstand voneinander aufweisen, wie z.B. zwei Zimmer in zwei unterschiedlichen Abteilungen eines Krankenhauses.

Zur Durchführung der Behandlung ist eine Bestrahlungsvorrichtung 20 vorgesehen, welche in diesem Ausführungsbeispiel eine Fixed-Beam-Vorrichtung ist, die an einer Wand des Untersuchungsraum 8' befestigt ist. Durch einen Strahlauslass der Bestrahlungsvorrichtung 20 wird ein Strahl 22 auf das zu behandelnde Gewebe des Patienten 2 gerichtet. Bei der Verwendung einer Fixed-Beam-Vorrichtung 20 ist die Richtung des Strahles 22 festgelegt und lediglich die Patientenliege 4 wird verstellt, um den Patienten 2 in genau die Stellung zu bringen, in der sein erkranktes Gewebe behandelt werden kann. Allerdings sind auch andere Ausführungen der Bestrahlungsvorrichtung 20 möglich, deren Beweglichkeit weniger eingeschränkt ist, so dass der Patient 2 durch Verstellen der Bestrahlungsvorrichtung 20 aus verschiedenen Richtungen bestrahlt werden kann.

Der bei der Untersuchung gewonnene Datensatz wird zum Verstellen der Bestrahlungsvorrichtung 20 und der Patientenliege 4' gegeneinander über eine Steuereinheit benutzt. Die Patientenliege 4', falls sie eine zweite separate Patientenliege 4' ist, wird mit der Positionsmechanik 12' in die für die Bestrahlung erforderliche Position verstellt.

Beim Erstellen eines Bestrahlungsplans wird außerdem die gespeicherte Information über die Liegefläche 10 benutzt. Da die beiden Patientenliegen 4 und 4' gleich oder gar dieselben sind, muss die Liegefläche 10, 10' in der Strahlentherapieplanung nicht eliminiert werden, sondern die durch ein Bild gebendes Verfahren erstellten Aufnahmen erhaltenen Daten werden weiter verwendet, was zu einer wesentlichen Vereinfachung der Behandlung, führt.

## Patentansprüche

1. Verfahren zur Untersuchung und Behandlung eines Patienten (2), bei dem
a) der Patient (2) zu einer Untersuchung auf eine erste Patientenliege (4) positioniert wird, wo
b) eine Untersuchung eines zu behandelnden Gewebes mit einem Bild gebenden Untersuchungsgerät (6, 14), insbesondere mit einem Computertomographen (6), durchgeführt wird, und
c) der Patient (2) zur Behandlung auf eine Patientenliege (4') positioniert wird, die eine zur ersten Patientenliege (4) identisch aufgebaute Liegefläche (10, 10') aufweist, wo
d) das zu behandelnde Gewebe mit einer Bestrahlungsvorrichtung (20) bestrahlt wird.

2. Verfahren nach Anspruch 1,
bei dem der Patient (2) zur Behandlung auf eine Patientenliege (4') positioniert wird, die eine zur ersten Patientenliege (4) identisch aufgebaute Positionsmechanik (12) aufweist.

3. Verfahren nach Anspruch 1 oder 2,
bei dem zur Untersuchung und zur Behandlung dieselbe Patientenliege (4, 4') verwendet wird.

4. Verfahren nach Anspruch 3,
bei dem nach der Untersuchung die Patientenliege (4, 4') zur Bestrahlungsvorrichtung hin verfahren wird.

5. Verfahren nach Anspruch 1 oder 2,
bei dem der Patient (2) zur Untersuchung auf die erste (4) und zur Behandlung auf eine zweite separate Patientenliege (4') positioniert wird.

6. Verfahren nach Anspruch 5,
bei dem die erste (4) und die zweite Patientenliege (4') jeweils über eine identische Positionsmechanik (12, 12') zur Untersuchung und zur Behandlung in gleichen Stellungen verstellt werden.

7. Verfahren nach Anspruch 6,
bei dem die räumliche Stellung der ersten Patientenliege (4) von einer Rechnereinheit in einem Datensatz erfasst wird und dieser Datensatz für eine automatisierte Einstellung der zweiten Patientenliege (4') über ihre Positionsmechanik (12') verwendet wird.

8. Verfahren nach einem der vorhergehenden Ansprüche,
bei dem die Behandlung mit der Bestrahlungsvorrichtung (14) in einem anderen Zimmer (8') als die Untersuchung mit dem Untersuchungsgerät (6) durchgeführt wird.

9. Verfahren nach einem der vorhergehenden Ansprüche,
bei dem die Liegefläche (10, 10') der Patientenliege (4, 4') in einen Bestrahlungsplan bei der Bestrahlung des zu behandelnden Gewebes einbezogen wird.

10. Patientenraum (8, 8') mit einer Untersuchungsstelle, an der ein Untersuchungsgerät (6, 14), insbesondere ein Computertomograph (6) aufgestellt ist, und einer Behandlungsstelle, an der eine Bestrahlungsvorrichtung (20) aufgestellt ist, wobei an dem Untersuchungsgerät (6, 14) und an der Bestrahlungsvorrichtung (20) Patientenliegen (4, 4') mit identisch aufgebauten Liegeflächen (10, 10') positionierbar sind.

11. Patientenraum (8, 8') nach Anspruch 10, wobei eine Patientenliege (4, 4') eine Positionsmechanik (12, 12') aufweist, mit deren Hilfe die Patientenliege (4, 4') vom Untersuchungsgerät (6, 14) zur Behandlungsvorrichtung (20) bewegbar ist.

12. Untersuchungsgerät (6, 14) zur Durchführung einer medizinischen Untersuchung eines Patienten (2), an dem eine Patientenliege (4, 4') positionierbar ist, deren Liegefläche (10, 10') zur Positionierung an einer Bestrahlungsvorrichtung (20) ausgebildet ist.
